(19) Europäisches Patentamt
European Patent Office
Office européen des brevets

(11) Veröffentlichungsnummer : **0 067 964**
**B1**

(12) **EUROPÄISCHE PATENTSCHRIFT**

(45) Veröffentlichungstag der Patentschrift : **03.04.85**

(21) Anmeldenummer : **82104366.8**

(22) Anmeldetag : **18.05.82**

(51) Int. Cl.[4] : **C 07 D493/04**// A61K31/34 ,(C07D493/04, 307:00, 307:00)

# (54) Verfahren zur Herstellung von Isosorbid-2-nitrat.

(30) Priorität : **22.06.81 DE 3124410**

(43) Veröffentlichungstag der Anmeldung : **29.12.82 Patentblatt 82/52**

(45) Bekanntmachung des Hinweises auf die Patenterteilung : **03.04.85 Patentblatt 85/14**

(84) Benannte Vertragsstaaten : **AT BE CH DE FR GB IT LI LU NL**

(56) Entgegenhaltungen :
**EP-A- 0 045 076**
**EP-A- 0 059 664**
**EP-A- 0 578 471**
**DE-A- 2 903 983**
**US-A- 4 065 488**

(73) Patentinhaber : **Heinrich Mack Nachf.**
**Postfach 140**
**D-7918 Illertissen (DE)**

(72) Erfinder : **Stoss, Peter, Dr.**
**Mozartstrasse 15**
**D-7918 Illertissen (DE)**

(74) Vertreter : **Lederer, Franz, Dr. et al**
**Patentanwälte Dr. Franz Lederer Reiner F. Meyer**
**Lucile-Grahn-Strasse 22**
**D-8000 München 80 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 067 964 B1

**Beschreibung**

Die Verwendung organischer Nitrate bei koronaren Erkrankungen gehört zu den heute allgemein anerkannten Therapieprinzipien. Am Beginn stand die Beobachtung, daß man mit Nitroglycerin einen Anfall von Angina pectoris kupieren kann. Später ging man daran, diese Therapie zu einer Prophylaxe zu erweitern. Voraussetzung dafür waren Substanzen mit ähnlicher Wirkung, jedoch längerer Wirkungsdauer. Unter der Vielzahl der nach diesem Gesichtspunkt synthetisierten und geprüften Substanzen hat sich das Isosorbid-dinitrat (ISDN) am besten durchgesetzt und nimmt heute einen festen Platz unter den Arzneiverordnungen ein. Aber auch in diesem Fall lassen sich befriedigende Verabreichungsintervalle nur durch besondere galenische Formulierungen mit Retardwirkung erzielen. Es besteht deshalb weiterhin ein berechtigtes Bedürfnis nach Nitraten mit substanzbedingter längerer Wirkungsdauer.

Als Ursache der kurzen Halbwertszeit von ISDN wurde die rasche enzymatische Biotransformation in der Leber erkannt. Bei der Metabolisierung entstehen hauptsächlich Isosorbid-2-nitrat (2-ISM), Isosorbid-5-nitrat (5-ISM), Isosorbid (IS), sowie entsprechende Konjugate [S. F. Sisenwine und H. W. Ruelius, J. Pharmacol. Exp. Ther. 176, 269 (1971)].

R. L. Wendt, J. Pharmacol. Exp. Ther. 180, 732 (1972) erbrachte den Nachweis der gleichartigen, nitrattypischen Wirkung von 2-ISM und 5-ISM. Weitere Untersuchungen ergaben wesentliche therapeutische Vorteile dieser Metaboliten gegenüber der Muttersubstanz, insbesondere eine verbesserte Resorption und damit eine höhere Bioverfügbarkeit sowie eine deutlich längere Halbwertszeit. Mithin kann eine Retardierung zur Erzielung eines Langzeiteffekts entbehrlich werden. Welchem der beiden Isosorbidmononitrate der Vorzug zu geben ist, wird von der Art der medizinischen Notwendigkeiten abhängen. So sind Anwendungsbereiche denkbar, bei denen die Verabreichung von 2-ISM vorteilhaft sein kann. Der direkten Anwendung der Mononitrate, insbesondere von 2-ISM steht jedoch bisher die aufwendige und sehr teure Synthese dieser Verbindung entgegen.

Nach I. G. Csizmadia und L. D. Hayward, Photochem. Photobiol. 4, 657 (1965) wird 2-ISM durch partielle Nitrierung von Isosorbid hergestellt. Das hierbei anfallende Gemisch enthält jedoch 2-ISM nur als untergeordneten Bestandteil, neben 5-ISM, ISDN und IS. Aus diesem Gemisch kann 2-ISM durch Säulenchromatographie gewonnen werden. Wegen der geringen Ausbeuten sowie der zeitraubenden und teuren Isolierungsmethode kommt diesem Weg keine praktische Bedeutung zu.

Auch eine weitere Methode [M. Anteunis et al., Org. Magnet. Resonance 3, 693 (1971)], wonach IS, in bekannter Weise, zunächst in das ISDN überführt und dieses nachfolgend partiell hydrolysiert wird, ergibt wiederum die schon erwähnten Gemische aus ISDN, 2-ISM, 5-ISM und IS, deren Trennung und Isolierung nicht auf wirtschaftlich vertretbare Weise durchführbar ist.

In der DOS 2 751 934 und in dem korrespondierenden US- Patent 4 056 488 ist folgendes Verfahren beschrieben : IS wird, in Gegenwart eines sauren Katalysators, mittels eines Säure-halogenids oder -anhydrids zu einer Mischung aus IS, IS-2-acylat, IS-5-acylat und IS-2,5-diacylat acyliert. Daraus extrahiert man IS, um in der nachfolgenden Nitrierungsstufe die Bildung des als explosionsgefährlich bekannten ISDN zu verhindern. Das verbleibende Gemisch aus IS-2-acylat, IS-5-acylat und IS-2,5-diacylat wird mit Salpetersäure verestert, unter Entstehung einer Mischung von IS-2-acylat-5-nitrat, IS-5-acylat-2-nitrat und IS-2,5-diacylat. Durch partielle Hydrolyse erhält man daraus ein Gemisch aus 2-ISM, 5-ISM und IS. Letzteres muß wiederum durch Extraktion entfernt werden, ehe aus dem verbleibenden Rückstand durch Umkristallisieren aus geeigneten Lösungsmitteln 2-ISM isoliert wird. Auch nach dieser Methode entsteht das gewünschte 2-ISM nur in sehr mäßiger Ausbeute (24 % der Theorie). Die in erheblicher Menge gebildeten, oben erwähnten Begleitprodukte, entziehen einen großen Teil des eingesetzten Isosorbids der erwünschten Reaktion und verursachen somit das insgesamt unbefriedigende Ergebnis.

Alle vorgenannten Verfahren sind dadurch gekennzeichnet, daß keine selektive Herstellung von 2-ISM möglich ist, sondern stets Gemische anfallen, die nachfolgend durch geeignete Trennverfahren in die einzelnen Bestandteile zerlegt werden müssen. Diese Vorgehensweise ist aufwendig und teuer. Sie gestattet die Isolierung von 2-ISM jeweils nur in geringen Ausbeuten und läßt damit eine wirtschaftliche Herstellung nicht zu.

Das erste Verfahren zur selektiven Herstellung von 2-ISM wurde in der DOS 2 903 983 bekanntgemacht. Danach wird Isomannid mittels eines Halogenids oder des Anhydrids der Trifluormethansulfonsäure zu Isomannid-2-trifluormethansulfonat umgesetzt. Dessen Reaktion mit einem Alkali-, Erdalkali- oder organischem Nitrat liefert, unter Konfigurationsumkehr am Kohlenstoff-Atom 2 des Ringsystems, das gewünschte Endprodukt.

Dieses Verfahren besitzt gegenüber den früher beschriebenen zweifellos den Vorteil, in einer eindeutigen Folge von nur zwei Reaktionsschritten, zu einem definierten Produkt zu führen. Dennoch läßt es hinsichtlich der Wirtschaftlichkeit sehr zu wünschen übrig. So ist der als Ausgangsprodukt eingesetzte Isomannid derzeit wesentlich schwerer zugänglich und auch erheblich teurer als Isosorbid. Auch die zur Umsetzung benötigten Chemikalien müssen zu den ausgesprochen teuren gerechnet werden. Schließlich liegt die nach den angegebenen Beispielen zu erzielende Gesamtausbeute nur bei etwa 13 % der Theorie. Für ein technisches Verfahren dürfte dieser Weg deshalb ebenfalls nicht infrage kommen.

Es besteht somit nach wie vor ein Bedürfnis nach Herstellungsverfahren für 2-ISM, die auf leicht zugänglichen Ausgangsprodukten basieren und unter Verwendung preiswerter Chemikalien, mit besseren Ausbeuten als nach dem Stand der Technik möglich, die gewünschte Verbindung liefern.

Ein derartiges vorteilhaftes Verfahren zur Herstellung von Isosorbid-2-nitrat ist Gegenstand dieser Erfindung.

Bekanntlich kommen für die Acylierung von Isosorbid im Wesentlichen folgende Methoden in Betracht :

— Veresterung mit Säuren
— Umesterung mit Säure-estern
— Acylierung mit Acylhalogeniden
— Acylierung mit Anhydriden

Als Diol bietet IS zwei Angriffsmöglichkeiten. Demgemäß erhält man bei Acylierungsversuchen stets Gemische aus IS, IS-2-acylat, IS-5-acylat und IS-2,5-diacylat. Die Zusammensetzung des Produktgemisches schwankt dabei in Abhängigkeit von der Herstellungsmethode und den Reaktionsbedingungen. In der Regel findet man jedoch die vier möglichen Bestandteile etwa zu gleichen Anteilen. Über geringfügige Verschiebungen der Produktverteilung wurde in der Literatur berichtet. Systematische Untersuchungen darüber liegen bisher noch nicht vor.

So wird z. B. in der schon erwähnten DOS 2 751 934 ein durch Acetylierung von IS mit Essigsäureanhydrid, in Gegenwart saurer Katalysatoren erhaltenes Gemisch der vier möglichen Bestandteile, mit nicht näher mitgeteilter Zusammensetzung beschrieben. Aus den Folgereaktionen und der Ausbeute an Endprodukt läßt sich schließen, daß IS-2-acetat/IS-5-acetat etwa im Verhältnis 1 : 2 vorgelegen haben, neben größeren Mengen an IS und IS-2,5-diacetat.

Über die Abhängigkeit der Produktverteilung von den Reaktionsbedingungen berichten K. W. Buck et al., Carbohydrate Res. 2, 122 (1966). Bei der Umsetzung von IS mit 1 mol Acetanhydrid in Pyridin erhielten diese Autoren, neben IS-2,5-diacetat als Hauptprodukt, 2- und 5-Monoacetat im Verhältnis von 1,7 : 1. In Gegenwart von Pyridinhydrochlorid wurde dagegen eine Verteilung 2-Acetat/5-Acetat von 1 : 3,6 erhalten, jedoch insgesamt wieder nur in untergeordneter Menge, neben 2,5-Diacetat.

H. Matyschok und S. Ropuszynski, Chem. Abstr. 81, 3311 (1974), beschreiben die Veresterung von IS mit Essig-, Propion- und Buttersäure, in Gegenwart eines stark sauren Ionenaustauschers, wobei Mono- und Diacyl-Verbindungen erhalten werden. Allerdings wird in der zitierten Arbeit nicht zwischen den 2- und 5-Acylaten differenziert, sondern nur die Isolierung von « Monoacylaten », in relativ bescheidenen Ausbeuten erwähnt. Auch nähere Angaben zur Identifizierung und Überprüfung der Reinheit der hergestellten Substanzen fehlen. Es kann davon ausgegangen werden, daß auch in diesem Fall Gemische vorgelegen haben. Eigene Nacharbeitungen der angegebenen Versuchsdurchführung bestätigen diese Annahme.

Das erfindungsgemäße Verfahren schafft erstmals die Möglichkeit, die Acylierung von IS mit hoher Regioselektivität zum IS-5-acylat zu lenken, ohne daß nennenswerte Anteile des isomeren IS-2-acylats entstehen. Dies bietet die Voraussetzung dafür, daß im weiteren Reaktionsverlauf durch Nitrierung und Umesterung, praktisch isomerenfreies 2-ISM hergestellt werden kann. Die Reaktionsführung gemäß der Erfindung ermöglicht gleichzeitig die Isolierung des reinen 2-ISM in sehr viel besseren Ausbeuten, als das nach den bisher bekannten Verfahren möglich war. Damit wird zum ersten Mal die Herstellung von 2-ISM auch unter wirtschaftlichen Gesichtspunkten interessant.

Die Erfindung betrifft somit ein Verfahren zur Herstellung von 2-ISM, bestehend aus folgenden Stufen :

— Acylierung von IS, wobei der Eintritt der Acylgruppe mit hoher Regioselektivität in 5-Stellung erfolgt und man zu praktisch isomerenfreiem IS-5-acylat gelangt,

— Veresterung dieses isomerenfreien IS-5-acylats mit Salpetersäure, unter Bildung von IS-5-acylat-2-nitrat und

— Abspaltung des Acylrestes durch Umesterung

Das erfindungsgemäße Verfahren wird wie folgt durchgeführt :

Man acyliert IS mit 1 bis 2 Moläquivalenten eines Carbonsäureanhydrids, in Gegenwart von 0,005 bis 0,02 Moläquivaltenten eines Katalysators. Die Acylierung erfolgt entweder ohne Lösungsmittel oder in Gegenwart eines inerten Lösungsmittels. Als solche können eingesetzt werden : die den entsprechenden Acylrest enthaltenden Carbonsäuren, wie Essigsäure, Propionsäure oder Buttersäure, ferner Chloroform, Dichlormethan, Aceton, Ethyl-methyl-keton, Dioxan, Tetrahydrofuran, Acetonitril, Dimethylformamid, Dimethylacetamid, Dimethylsulfoxid oder Ethylenglykol-dialkylether. Die Reaktion wird in einem Temperaturbereich von − 50 °C bis + 100 °C durchgeführt, vorzugsweise im Bereich von 0 °C bis 30 °C und erfordert Reaktionszeiten von wenigen Minuten bis zu hundert Stunden, wobei 10 bis 30 Stunden bevorzugt werden.

Als Katalysatoren können eingesetzt werden : Salze von Metallen aus der 2., 3., 4., 5. und 8. Gruppe des Periodensystems, wobei die Aktivität innerhalb einer Gruppe im allgemeinen von der 1. zur 6. Periode zunimmt. Die wirksamsten Katalysatoren sind deshalb vorwiegend in der 6. Periode anzutreffen. Beispielhaft seien erwähnt : Salze von Calcium, Strontium, Barium, Zink, Cadmium, Quecksilber Indium, Thallium, der Lanthaniden, Zinn, Blei, Antimon, Wismut, Eisen, Cobalt, Nickel. Bevorzugt werden Salze

von Barium, Quecksilber, Blei und Wismut, insbesondere Blei. Dabei können die zur Salzbildung herangezogenen Säuren in weiten Bereichen variiert werden und umfassen Kohlensäure, aliphatische und aromatische Carbonsäuren ebenso wie anorganische Säuren. Als Beispiele derartiger Metallsalze seien erwähnt : Carbonate, Acetate, Propionate, Benzoate, Chloride, Nitrate und Phosphate.

Unter Acylresten, die mittels des Carbonsäureanhydrids eingeführt werden, sind zu verstehen :

— geradkettige und verzweigte aliphatische Carbonsäurereste mit 2 bis 6 Kohlenstoffatomen, wie Acetyl, Propionyl, Butyryl, Valeryl, Capronyl, Isobutyryl, Isovaleryl und Pivaloyl,

— cycloaliphatische Carbonsäurereste mit 6 bis 8 Kohlenstoffatomen, wie Cyclopentancarbonyl, Cyclohexancarbonyl und Cycloheptancarbonyl,

— unsubstituierte und substituierte Benzolsäurereste, wobei die Substituenten niedere Alkylgruppen, Halogen-, Methoxy- und Nitrogruppen umfassen, wie Benzoyl, o-, m- und p-Toluoyl, Dimethylbenzoyl, Trimethyl-benzoyl, Methoxy-benzoyl, Nitro-benzoyl und Halogen-benzoyl.

Die Acylierung von IS nach dem erfindungsgemäßen Verfahren erfolgt mit hoher Regioselektivität in Bezug auf IS-5-acylat. Dabei erreicht man nicht nur, wie nach dem Stand der Technik für die Acetate angegeben, ein Produktverhältnis IS-5-acetat/IS-2-acetat von ca. 2 : 1, beziehungsweise 3,6 : 1, neben größeren Mengen IS und IS-2,5-diacetat. Es gelingt vielmehr eine nahezu quantitative Umsetzung des Isosorbids zum IS-5-acylat, wobei der Anteil des isomeren IS-2-acylats unterhalb der dünnschichtchromatografischen Nachweisgrenze liegt und auch IS-2,5-diacetat nur in unbedeutender Menge entsteht. In der Praxis läßt sich, bei optimaler Reaktionsführung, ein Produkt mit nachfolgender Zusammensetzung erreichen : ca. 94-96 % IS-5-acylat, ca. 3-6 % IS-2,5-diacylat, unter 1 % IS ; IS-2-acylat ist nicht nachweisbar.

Falls gewünscht, kann aus diesem Rohprodukt, mittels geeigneter Aufarbeitungs- und Reinigungsmethoden, wie z. B. Extraktion, Destillation oder Umkristallisation, IS-5-acylat in über 99 %iger Reinheit gewonnen werden. Für die Folgereaktionen ist sowohl das reine, über 99 %ige IS-5-acylat, als auch das bei der selektiven Acylierung anfallende rohe IS-5-acylat, mit einem Gehalt von etwa 94-96 %, ohne Isolierung und Reinigung direkt einsetzbar.

Die Fortführung des Verfahrens besteht darin, das auf diesem wirtschaftlichen Weg erstmals in beliebigen Mengen selektiv zugängliche IS-5-acylat, in an sich bekannter Weise, mit Salpetersäure zu verestern, wobei IS-5-acylat-2-nitrat erhalten wird, welches nicht durch isomeres IS-2-acylat-5-nitrat und auch nicht durch ISDN verunreinigt ist. In der nächsten Stufe wird schließlich durch Umesterung, in an sich bekannter Weise, die 5-Acylgruppe abgespalten, wobei 2-ISM entsteht.

Die Reaktionsführung kann dabei sowohl derart erfolgen, daß die auftretenden Zwischenprodukte isoliert und in dieser Form für die nächste Stufe eingesetzt werden. Zweckmäßigerweise verzichtet man jedoch auf die Isolierung der intermediär gebildeten Stoffe und verarbeitet diese in der anfallenden Form direkt weiter. Man gelangt so, auf technisch wenig aufwendige Weise, zu 2-ISM, das durch einfaches Umkristallisieren des Rohprodukts aus einem geeigneten Lösungsmittel in reiner Form und mit hoher Ausbeute zu erhalten ist.

IS-5-acylat-2-nitrate sind bereits in der DOS 2 221 080 beschrieben. Sie dienen jedoch dort nicht zur Herstellung des 2-ISM, sondern werden vielmehr aus diesem synthetisiert. Zwar wird auch auf die umgekehrte Möglichkeit hingewiesen. Nach dem Stand der Technik ist dies aber nur über chromatografische Trennverfahren von Acylierungsgemischen realisierbar und wurde in der Praxis bisher auch nicht durchgeführt.

Nach der DOS 2 751 934 wird IS-5-acylat-2-nitrat mit einer anorganischen Base, wie Natrium- oder Kalium-hydroxid oder -carbonat hydrolysiert. Gemäß der angegebenen Verfahrensweise kann jedoch reines IS-5-acylat-2-nitrat wiederum nur durch extraktive oder chromatografische Reinigungsschritte aus den vorhandenen Gemischen abgetrennt werden. In der bevorzugten Ausführungsform verzichtet man darauf und unterwirft das Gemisch der Hydrolyse. Vorteilhafter läßt sich die Abspaltung der 5-Acylgruppe erreichen, indem man in ebenfalls bekannter Weise, z. B. in Anlehnung an die DAS 2 903 927, IS-5-acylat-2-nitrat in einem niederen Alkohol, in Gegenwart eines Alkalialkoholats, einer Umesterung unterwirft. Als niedere Alkohole kommen vorzugsweise Methanol oder Ethanol infrage und als Alkoholate bevorzugt Natrium- oder Kalium-alkoholate. Gegenüber der wäßrig-alkalischen Hydrolyse bietet die Umesterung den Vorteil der größeren Selektivität und Reaktionsgeschwindigkeit. Darüber hinaus entfällt der Neutralisationsaufwand und es genügen katalytische Mengen des Alkoholats. Auch das Lösungsmittelvolumen kann wesentlich geringer gehalten werden.

Insgesamt gelingt es, mit Hilfe des erfindungsgemäßen Verfahrens, IS mit hoher Selektivität, über die Stufen der Acylierung, Nitrierung und Umesterung in 2-ISM zu überführen. Dabei wird der eingesetzte IS, im Gegensatz zu den bisher bekannten Verfahren, nahezu vollständig in die erwünschte Reaktionsfolge einbezogen, so daß keine Ausbeuteverminderung durch Nebenreaktionen eintritt. Dies ermöglicht die Herstellung von 2-ISM, ausgehend von preiswertem IS, in drei Reaktionsschritten, wobei auf die Isolierung von Zwischenprodukten gegebenenfalls verzichtet werden kann. Das gewünschte Produkt entsteht in hohen Ausbeuten und vorzüglicher Reinheit. Damit wird 2-ISM für den Einsatz als pharmazeutischer Wirkstoff, sowohl unter dem Gesichtspunkt der Wirtschaftlichkeit, als auch der Qualität, im technischen Maßstab verfügbar.

Die nachfolgenden Beispiele dienen der Erläuterung der Erfindung :

Beispiel 1

a) Eine Mischung von 146 g Isosorbid, 305 g p-Toluoylsäureanhydrid und 1 l Dichlormethan wird mit 5 g Bleiacetat versetzt und 40 Stunden bei Raumtemperatur gerührt. Dabei geht zunächst alles in Lösung, später fällt das Reaktionsprodukt aus. Man kühlt auf 0 °C ab, saugt den kristallinen Niederschlag ab und kristallisiert aus Toluol um. Man erhält 210 g 5-(p-Toluoyl)-isosorbid ; Schmp. 160-162 °C.

b) Aus 130 g 65-proz. Salpetersäure und 400 ml Essigsäureanhydrid wird unter Kühlung bei ca. 15 °C eine Nitriermischung bereitet. Dazu gibt man zunächst 100 ml Dichlormethan und danach portionsweise 264 g 5-(p-Toluoyl)-isosorbid, wobei die Temperatur durch Kühlung bei 10-15 °C gehalten wird. Anschließend erwärmt man das Gemisch auf 25 °C und beläßt es bei dieser Temperatur, bis im DC eine vollständige Umsetzung erkennbar ist (ca. 1 Stunde). Man versetzt mit 400 ml Dichlormethan und 1 l Wasser. Die wäßrige Schicht wird verworfen, die organische Phase mit verdünnter wäßriger Ammoniak-Lösung ausgerührt, abgetrennt und im Vakuum eingeengt. Der Rückstand wird aus Methanol umkristallisiert. Ausbeute : 290 g 5-(p-Toluoyl)-isosorbid-2-nitrat ; Schmp. 93-94 °C.

c) 309 g 5-(p-Toluoyl)-isosorbid-2-nitrat werden in 1 l Methanol suspendiert. Unter Rühren tropft man 30-proz. methanolische Natriummethylat-Lösung bis zur deutlich alkalischen Reaktion dazu, wobei eine klare Lösung entsteht. Die Mischung wird 2 Stunden bei 40 °C gerührt, danach mit Essigsäure neutralisiert und im Vakuum vom Lösungsmittel befreit. Den Rückstand verteilt man bei 40 °C zwischen 1 l Wasser und 500 ml Petroläther, trennt die Wasserphase ab und engt diese auf ca. 300 ml ein. Beim Abkühlen auf 0 °C kristallisiert Isosorbid-2-nitrat aus. Man filtriert ab und wäscht mit 100 ml kaltem Isopropanol nach. Ausbeute : 151 g ; Schmp. 53-55 °C.

Beispiel 2

Eine Mischung aus 146 g Isosorbid, 150 g Essigsäure-anhydrid und 5 g Bleiacetat wird 20 Stunden bei Raumtemperatur stehen gelassen. Aus 350 ml Essigsäure-anhydrid und 130 g 65-proz. Salpetersäure bereitet man unter Kühlung bei ca. 15 °C eine Nitriermischung. Unter Rühren und Kühlung auf 15 °C, gibt man das vorstehende Acylierungsgemisch dazu. Man rührt noch 2 Stunden bei Raumtemperatur und versetzt danach mit 300 ml Dichlormethan und 1 l Wasser. Die wäßrige Phase wird verworfen, die organische Phase mit verdünnter wäßriger Ammoniaklösung gewaschen und im Vakuum eingeengt. Den Rückstand nimmt man in 200 ml Methanol auf, versetzt mit 10 ml 30-proz. methanolischer Natriummethylat-Lösung und rührt 1 Stunde. Danach neutralisiert man das Gemisch mit Essigsäure und entfernt das Lösungsmittel im Vakuum. Der Rückstand wird unter leichtem Erwärmen (20-30 °C) in 200 ml Wasser gelöst, die Lösung mit Aktivkohle ausgerührt, filtriert und auf 0 °C abgekühlt. Das auskristallisierte Isosorbid-2-nitrat wird abgesaugt und mit 100 ml eiskaltem Isopropanol gewaschen. Ausbeute : 151 g ; Schmp. 53-55 °C.

Beispiel 3

Auf gleiche Weise, wie im Beispiel 2 beschrieben, erhält man durch Acylierung von Isosorbid mit den nachfolgend aufgeführten Carbonsäure-anhydriden die angegebenen Isosorbid-5-acylate, die ebenfalls, ohne Isolierung, mit Salpetersäure verestert und durch nachfolgende Umesterung mit Methanol mit vergleichbaren Ausbeuten in Isosorbid-2-nitrat vom Schmp. 53-55 °C überführt werden :

Flüssige Anhydride werden dabei ohne Lösungsmittel umgesetzt, feste Anhydride in einem geeigneten Lösungsmittel, wie z. B. Dichlormethan, Tetrahydrofuran, Dioxan, Acetonitril oder Dimethylformamid gelöst.

| Eingesetztes Anhydrid | Isosorbid-5-acylate |
|---|---|
| Propionsäure- | 5-Propionyl-isosorbid |
| Buttersäure- | 5-Butyryl-isosorbid |
| Valeriansäure- | 5-Valeryl-isosorbid |
| Capronsäure- | 5-Capronyl-isosorbid |
| Isobuttersäure- | 5-Isobutyryl-isosorbid |
| Isovaleriansäure- | 5-Isovaleryl-isosorbid |
| Pivalinsäure- | 5-Pivaloyl-isosorbid |
| Cyclopentancarbonsäure- | 5-Cyclopentancarbonyl-isosorbid |
| Cyclohexancarbonsäure- | 5-Cyclohexancarbonyl-isosorbid |
| Cycloheptancarbonsäure- | 5-Cycloheptancarbonyl-isosorbid |
| Benzoesäure- | 5-Benzoyl-isosorbid |
| o-Toluoylsäure- | 5-(o-Toluoyl)-isosorbid |
| p-Chlorbenzoesäure- | 5-(p-Chlorbenzoyl)-isosorbid |
| m-Methoxy-benzoesäure- | 5-(m-Methoxybenzoyl)-isosorbid |
| p-Nitro-benzoesäure- | 5-(p-Nitrobenzoyl)-isosorbid |

**Ansprüche**

1. Verfahren zur Herstellung von Isosorbid-2-nitrat der Formel

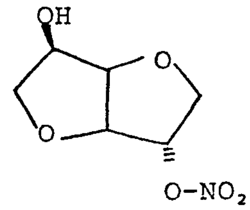

durch Acylierung von Isosorbid mit einem Carbonsäureanhydrid in Gegenwart eines Katalysators zu Isosorbid-5-acylat, Nitrierung dieses Isosorbid-5-acylates zu Isosorbid-5-acylat-2-nitrat und anschließende Umwandlung dieses Isosorbid-5-acylat-2-nitrates zu Isosorbid-2-nitrat, dadurch gekennzeichnet, daß man

a) als Carbonsäureanhydrid ein Carbonsäureanhydrid von geradkettigen oder verzweigtkettigen, aliphatischen Carbonsäuren mit 2 bis 6 Kohlenstoffatomen, von cycloaliphatischen Carbonsäuren mit 6 bis 8 Kohlenstoffatomen oder von unsubstituierter oder substituierter Benzoesäure verwendet und bei der Acylierung des Isosorbids mit 1 bis 2 Moläquivalenten des Carbonsäureanhydrids 0,005 bis 0,02 Moläquivalente eines Metallsalzes von Metallen der 2., 3., 4., 5. oder 8. Gruppe des Periodensystems als Katalysator unter Bildung von Isosorbid-5-acylat mit hoher Selektivität von wenigstens 90 % verwendet,

b) das so erhaltene Isosorbid-5-acylat in an sich bekannter Weise mit Salpetersäure zu Isosorbid-5-acylat-2-nitrat verestert, und

c) aus dem so erhaltenen Isosorbid-5-acylat-2-nitrat in an sich bekannter Weise durch partielle Umesterung mit einem niederen Alkohol in Gegenwart eines Alkalimetallalkoholates Isosorbid-2-nitrat freisetzt.

2. Verfahren nach Anspruch 1, dadurch gekennzeichnet, daß man in der Stufe a) als Metallsalz ein Carbonat, basisches Carbonat, Chlorid, Nitrat, Phosphat oder ein Salz einer aliphatischen oder aromatischen Carbonsäure verwendet.

3. Verfahren nach Anspruch 2, dadurch gekennzeichnet, daß man als Metallsalz einer aliphatischen oder aromatischen Carbonsäure ein Salz verwendet, dessen Acylrest dem Acylrest des zur Acylierung verwendeten Carbonsäureanhydrids entspricht.

4. Verfahren nach einem der vorgehenden Ansprüche, dadurch gekennzeichnet, daß man als Benzoesäureanhydrid Benzoesäureanhydrid, Toluoylsäureanhydrid, Dimethylbenzoesäureanhydrid, Trimethylbenzoesäureanhydrid, Methoxybenzoesäureanhydrid, Nitrobenzoesäureanhydrid oder Halogenbenzoesäureanhydrid verwendet.

5. Verfahren nach einem der vorhergehenden Ansprüche, dadurch gekennzeichnet, daß man als Metallsalze Salze von Calcium, Strontium, Barium, Zink, Cadmium, Quecksilber, Indium, Thallium, Lanthaniden, Zinn, Blei, Wismut, Antimon, Eisen, Kobalt oder Nickel verwendet.

6. Verfahren nach einem der vorhergehenden Ansprüche, dadurch gekennzeichnet, daß man das in Stufe a) hergestellte Isosorbid-5-acylat vor der weiteren Umsetzung durch Extraktion, Destillation oder Umkristallisation reinigt.

7. Verfahren nach einem der vorhergehenden Ansprüche, dadurch gekennzeichnet, daß man in Stufe c) die Umesterung mit Methanol oder Ethanol in Gegenwart des entsprechenden Natrium- oder Kaliumalkoholates durchführt.

**Claims**

1. Process for the preparation of isosorbide-2-nitrate of the formula

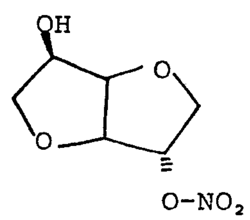

by acylation of isosorbide with a carboxylic acid anhydride in the presence of a catalyst to isosorbide-5-acylate, nitration of this isosorbide-5-acylate to isosorbide-5-acylate-2-nitrate and subsequent conversion of this isosorbide-5-acylate-2-nitrate to isosorbide-2-nitrate, characterized in that

a) as carboxylic acid anhydride there is used a carboxylic acid anhydride of straight-chained or

branch-chained aliphatic carboxylic acids having 2 to 6 carbon atoms, of cycloaliphatic carboxylic acids having 6 to 8 carbon atoms or of unsubstituted or substituted benzoic acid and in the acylation of the isosorbide with 1 to 2 mole equivalents of the carboxylic acid anhydride 0,005 to 0,02 mole equivalents of a metal salt of metals of group 2, 3, 4, 5 or 8 of the periodic table are used as catalyst with the formation of isosorbide-5-acylate with a high selectivity of at least 90 %,

b) the obtained isosorbide-5-acylate is esterified in a manner known per se with nitric acid to isosorbide-5-acylate-2-nitrate and

c) isosorbide-2-nitrate is released from the obtained isosorbide-5-acylate-2-nitrate in a manner known per se by partial re-esterification with a lower alcohol in the presence of an alkali metal alkoxide.

2. Process according to claim 1, characterized in that a carbonate, basic carbonate, chloride, nitrate, phosphate or a salt of an aliphatic or aromatic carboxylic acid is used as metal salt in step a).

3. Process according to claim 2, characterized in that as said metal salt of an aliphatic or aromatic carboxylic acid, there is used a salt the acyl radical of which corresponds to the acyl radical of the carboxylic acid anhydride used for the acylation.

4. Process according to any one of the preceding claims, characterized in that there is used benzoic acid anhydride, toluoylic acid anhydride, dimethyl benzoic acid anhydride, trimethyl benzoic acid anhydride, methoxy benzoic acid anhydride, nitrobenzoic acid anhydride or halobenzoic acid anhydride as said benzoic acid anhydride.

5. Process according to any one of the preceding claims, characterized in that salts of calcium, strontium, barium, zinc, cadmium, mercury, indium, thallium, lanthanides, tin, lead, bismuth, antimony, iron, cobalt or nickel are used as said metal salts.

6. Process according to any one of the preceding claims, characterized in that the isosorbide-5-acylate prepared in step a) is purified prior to the further reaction by means of extraction, distillation or recrystallization.

7. Process according to any one of the preceding claims, characterized in that in step c) the re-esterification with methanol or ethanol is carried out in the presence of the corresponding sodium or potassium alkoxide.

**Revendications**

1. Procédé de production de 2-nitrate d'isosorbide de formule

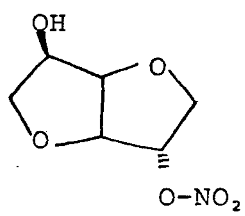

par acylation de l'isosorbide en 5-acylate d'isosorbide avec un anhydride d'acide carboxylique en présence d'un catalyseur, nitration de ce 5-acylate d'isosorbide en 5-acylate-2-nitrate d'isosorbide et transformation subséquente de ce 5-acylate-2-nitrate d'isosorbide en 2-nitrate d'isosorbide, caractérisé en ce que

a) on utilise comme anhydride d'acide carboxylique un anhydride d'un acide carboxylique aliphatique à chaîne droite ou ramifiée ayant 2 à 6 atomes de carbone, d'un acide carboxylique cycloaliphatique ayant 6 à 8 atomes de carbone ou d'acide benzoïque non substitué ou substitué et on utilise dans l'acylation de l'isosorbide avec 1 à 2 équivalents molaires de l'anhydride d'acide carboxylique, 0,005 à 0,02 équivalent molaire d'un sel métallique de métaux du 2e, 3e, 4e, 5e ou 8e groupe du Système Périodique comme catalyseur avec formation de 5-acylate d'isosorbide avec une haute sélectivité d'au moins 90 %,

b) on estérifie à l'acide nitrique d'une manière connue le 5-acylate d'isosorbide ainsi obtenu en 5-acylate-2-nitrate d'isosorbide, et

c) on libère le 2-nitrate d'isosorbide du 5-acylate-2-nitrate d'isosorbide ainsi obtenu d'une manière connue, par transestérification partielle avec un alcool inférieur en présence d'un alcoolate de métal alcalin.

2. Procédé suivant la revendication 1, caractérisé en ce qu'on utilise dans l'étape a) comme sel métallique, un carbonate, un carbonate basique, un chlorure, un nitrate, un phosphate ou un sel d'un acide carboxylique aliphatique ou aromatique.

3. Procédé suivant la revendication 2, caractérisé en ce qu'on utilise comme sel métallique d'un acide carboxylique aliphatique ou aromatique, un sel dont le reste acyle correspond au reste acyle de l'anhydride d'acide carboxylique utilisé pour l'acylation.

4. Procédé suivant l'une des revendications précédentes, caractérisé en ce qu'on utilise comme anhydride d'acide benzoïque, l'anhydride d'acide benzoïque, l'anhydride d'acide toluique, l'anhydride

d'acide diméthylbenzoïque, l'anhydride d'acide triméthylbenzoïque, l'anhydride d'acide méthoxybenzoïque, l'anhydride d'acide nitrobenzoïque ou un anhydride d'acide halogénobenzoïque.

5. Procédé suivant l'une des revendications précédentes, caractérisé en ce qu'on utilise comme sels métalliques, des sels de calcium, de strontium, de baryum, de zinc, de cadmium, de mercure, d'indium, de thallium, de lanthanides, d'étain, de plomb, de bismuth, d'antimoine, de fer, de cobalt ou de nickel.

6. Procédé suivant l'une des revendications précédentes, caractérisé en ce qu'on purifie le 5-acylate d'isosorbide préparé dans l'étape a) avant la réaction ultérieure par extraction, distillation ou recristallisation.

7. Procédé suivant l'une des revendications précédentes, caractérisé en ce qu'on conduit la transestérification dans l'étape c) avec du méthanol ou de l'éthanol en présence de l'alcoolate de sodium ou de potassium correspondant.